# EUROPEAN PATENT SPECIFICATION

(11) **EP 0 611 296 B1**
(45) Date of publication and mention of the grant of the patent: **18.03.1998**
(21) Application number: 92922748.6
(22) Date of filing: 03.11.1992
(51) Int. Cl.: A61B 17/36

(54) **METHOD OF THAWING CRYOSURGICAL APPARATUS**
VERFAHREN ZUM AUFTAUEN VON EINEM KRYOCHIRURGISCHEN INSTRUMENT
PROCEDE PERMETTANT DE DEGELER UN APPAREIL CRYOCHIRURGICAL

(30) Priority: 05.11.1991 GB 9123413
(43) Date of publication of application: 24.08.1994
(73) Proprietor: SPEMBLY CRYOSURGERY LIMITED, Andover, Hampshire SP10 4DR (GB)
(72) Inventor: CLARKE, Brian, Kevin, Roderick, Oakwood Derby DE2 2JL (GB)
(74) Representative: Turner, James Arthur
(86) International application number: PCT/GB92/02030
(87) International publication number: WO 93/08752

(56) References cited:
- WO-A-83/03961
- DE-A- 2 251 626
- US-A- 4 377 168
- Derwent's abstract, No 86-296 896/45, week 8645, ABSTRACT OF SU 1 217 377, (AS UKR LOW TEMP PHYSICS), 15 March 1986 (15.03.86)
- Derwent's abstract, No 84-86 863/14, week 8414, ABSTRACT OF SU 1 024 075, (ODESS MED INST), 23 June 1983 (23.06.83), abstract; figure 1

## Description

The invention concerns cryosurgical apparatus, and relates in particular to a method of thawing a cryosurgical probe subsequent to carrying out a freezing process, in order that the probe may be withdrawn.

Following an operation involving the destruction of tumourous tissue by freezing with a cooled probe inserted into the body, it is necessary to thaw or allow to be thawed the tissue immediately surrounding the probe in order that the latter may be withdrawn. Such thawing is commonly carried out by one of two principal methods. The first involves the use of latent body heat. This has the serious disadvantage that the process is extremely slow, thereby significantly increasing operation times. The second method utilises electrical heating of the probe tip. Although this is a considerably faster method, there are inherent safety problems which necessitate the utmost care in producing and using equipment of this kind.

The invention seeks to mitigate or obviate the above mentioned difficulties by providing a relatively fast yet safe method of raising the temperature of the probe tip on completion of an operation.

DE-A-2251626 describes a cryosurgical probe in which cryogen is supplied to a cooling tip through an innermost tube of a concentric arrangement, and the exhaust cryogen gas is removed through a second tube concentrically surrounding the first tube.

Reference is also made to WO-A-83/03961 in which the use of x-ray computer tomography is said to permit the monitoring of ice-ball growth using cryosurgery, thereby enabling the cryosurgery of deep-lying lesions and tumours, and the optimisation of cryotherapy of tissue close to the body surface and/or body cavities. This monitoring technique is used in conjunction with special cryoprobes designed to have an attenuation co-efficient as close as possible to that of biological tissue. The cryoprobes are constructed of low atomic number metals and/or plastics and can be used with liquid nitrogen or liquid noble gas, especially crypton and xenon.

Reference is also made to SU-A-1217377 which describes a Joule-Thomson gas cryosurgical instrument, and a method of thawing the instrument by using a gas of inversion temperature below the ambient temperature, for example, helium, neon or hydrogen. The gas is fed to the refrigerated head and, after passing through the Joule-Thomson throttle opening, the expanded gas becomes hotter, to speed up thawing of the probe.

According to one aspect of the invention there is provided a method of cooling and thawing cryosurgical apparatus having an internal circulation system for receiving cryogen, the internal circulation system including a first path and a second path, the method comprising:
supplying a liquid cryogen through the first path of the internal circulation system to a tip region of the apparatus to effect cooling thereof by vaporisation of the cryogen, and removing the cryogen through the second path of the internal circulation system;
interrupting the supply of liquid cryogen when surgery is complete; and passing an inert gas heated to a predetermined temperature through the internal circulation system to effect thawing of the apparatus; characterised in that:
   the inert gas circulated through the first and second paths is supplied to the internal circulation system through the first path, and is removed from the internal circulation system through the second path.

In a related aspect, the invention provides cryosurgical apparatus comprising:
a cryosurgical device having an internal circulation system for receiving cryogen, the internal circulation system including a first path and a second path;
liquid cryogen supply means for supplying liquid cryogen through the first path of the internal circulation system to a tip region of the device to cool the tip region by vaporisation of the liquid cryogen;
inert gas supply means for supplying to the internal circulation system, after the supply of cryogen has been interrupted at the end of surgery, a heated inert gas at a predetermined temperature to thaw the cryosurgical device; and
an exhaust line for receiving the cryogen exhaust through the second path of the internal circulation system; characterised in that:
   the inert gas supply means is coupled to supply the inert gas through the first path of the internal circulation system.

The invention is particularly suited to liquid nitrogen cooled probes, such as the kind in which the cryogen is delivered to the probe tip via a delivery tube located along the probe axis and returned via an annular gap within the probe housing surrounding the delivery tube.

Preferably the inert gas is nitrogen. This gas may be heated by feeding it to the apparatus via a heat exchanging arrangement.

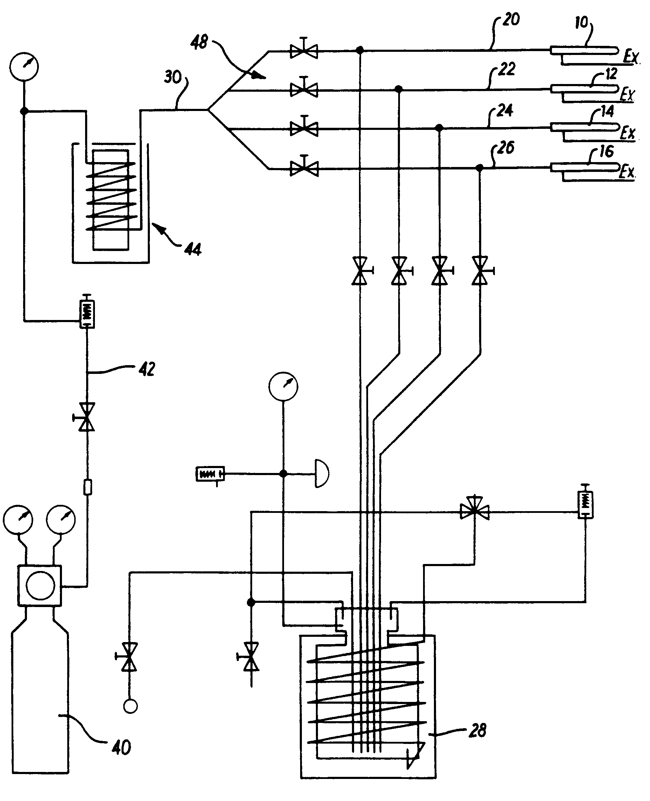

The invention will be described in more detail by way of example only with reference to the accompanying drawing which is a schematic block diagram showing a cryosurgical probe arrangement for utilising the thawing method of the invention.

The figure shows an arrangement containing four cryosurgical probes 10, 12, 14 and 16. Each probe is connected by a delivery line 20, 22, 24 and 26 to a supply vessel 28 of a liquid cryogen, such as liquid nitrogen.

The lines 20, 22, 24 and 26 are also in communication with a further delivery line 30 as described in more detail hereinafter.

The system provides a supply 40 of an inert gas, in the present example, nitrogen. Gas may be supplied through a delivery line 42 to a heat exchanging arrangement 44, and thus to the cryogen circulation systems of the probes 10, 12, 14 and 16 via the line 30 and the lines 20, 22, 24 and 26.

Each of the probes 10, 12, 14 and 16 may be supplied with the cryogen from the supply vessel 28 in any convenient manner, such as via a delivery tube located along the respective probe longitudinal axis, as is well known in the art. Following the freezing of the probe tip, the cryogen is then removed, for example via an annular gap within a housing of the probe surrounding the delivery tube, to a suitable exhaust line on the probe.

When the operation is complete and it is required to withdraw the probe, the supply of cryogen from the vessel 28 is shut off by means of a suitable valve arrangement. Gas is passed from the supply 40 through the line 42 to the heat exchanger 44. On passing through the heat exchanger, the gas is warmed to the required predetermined temperature. From the heat exchanger 44 the gas supply is passed through the line 30 and then via suitable valve systems 48 to the supply line of the appropriate probe. The warmed nitrogen is thereby circulated through the probe body and tip whereby it provides localised heat within the probe tip. The gas may then be exhausted through the existing probe cryogen outlet lines.

Any cryogen remaining within the probe circulation system is automatically purged by the incoming gas supply.

It will be appreciated that control and monitoring of the apparatus can be achieved by the incorporation of any appropriate valves, gauges, regulators or other instrumentation as is well known to the person skilled in the art. Such systems are not therefore described in detail herein.

There is therefore described a particularly convenient way of raising the temperature of the probe body. The method naturally permits suitable control of the speed of the thawing process by appropriate adjustments to the gas supply. The use of nitrogen gas in the given example also obviates problems of the formation of ice deposits within the cryogen circulation system on re-cooling which could arise if, for example, a liquid was used as the purging and heat transfer medium.

## Claims

1. A method of cooling and thawing cryosurgical apparatus (10, 12, 14, 16) having an internal circulation system for receiving cryogen, the internal circulation system including a first path and a second path, the method comprising:
supplying a liquid cryogen through the first path of the internal circulation system to a tip region of the apparatus to effect cooling thereof by vaporisation of the cryogen, and removing cryogen through the second path of the internal circulation system;
interrupting the supply of liquid cryogen when surgery is complete; and
passing an inert gas heated to a predetermined temperature through first and second paths of the internal circulation system to effect thawing of the apparatus; characterised in that:
the inert gas circulated through the first and second paths is supplied to the internal circulation system through the first path, and is removed from the internal circulation system through the second path.

2. A method according to claim 1 wherein the inert gas is nitrogen.

3. A method according to claim 1 or claim 2 wherein the inert gas is supplied to the apparatus via a heat exchanging arrangement (44).

4. A method according to any preceding claim, wherein the apparatus comprises a cryosurgical probe.

5. A method according to claim 4 wherein the inert gas is supplied to the probe tip via a delivery tube located along the probe axis and is returned via an annular gap within the probe housing surrounding the delivery tube.

6. A method according to claim 1, 2, 3, 4 or 5 wherein the cryogen is liquid nitrogen.

7. A method according to any preceding claim, wherein the cryosurgical apparatus comprises a plurality of cryosurgical probes (10, 12, 14, 16) each having a respective said internal circulation system with a first path and a second path, wherein the method comprises independently controlling, for each cryosurgical probe said supply of liquid cryogen to the internal circulation system of that probe, and independently controlling, for each cryosurgical probe after the supply of cryogen to that probe has been interrupted, said supply of heated inert gas through the internal circulation system of the probe.

8. Cryosurgical apparatus comprising:
a cryosurgical device (10, 12, 14, 16,) having an internal circulation system for receiving cryogen, the internal circulation system including a first path and a second path;
liquid cryogen supply means (28, 20, 22, 24, 26) for supplying liquid cryogen through the first path of the internal circulation system to a tip region of the device to cool the tip region by vaporisation of the liquid cryogen;
an exhaust line for receiving the cryogen exhaust through the second path of the internal circulation system; and:
inert gas supply means (40, 42, 44, 20, 22, 24, 26) for supplying to the internal circulation system, after the supply of cryogen has been interrupted at the end of surgery, a heated inert gas at a predetermined temperature to thaw the cryosurgical device; characterised in that:
the inert gas supply means is coupled to supply the inert gas through the first path of the internal circulation system.

9. Apparatus according to claim 8, wherein the inert gas supply means comprises a heat exchanger (44) for heating the inert gas as it is supplied from a source (40) of the inert gas to the cryosurgical device.

10. Apparatus according to claim 8 or 9 wherein the cryosurgical device comprises a cryosurgical probe (10,12,14,16).

11. Apparatus according to claim 8, 9 or 10, comprising a plurality of cryosurgical probes (10, 12, 14, 16,) each having an internal circulation system with a first path to which the liquid cryogen supply means and the inert gas supply means are coupled, and a second path to which a said exhaust line is coupled.

## Patentansprüche

1. Verfahren zum Kühlen und Auftauen einer kryochirurgischen Vorrichtung (10, 12, 1, 16) mit einem inneren Zirkulationssystem zur Aufnahme von kryogenem Stoff, wobei das innere Zirkulationssystem einen ersten Weg und einen zweiten Weg einschließt, indem man eine kryogene Flüssigkeit durch den ersten Weg des inneren Zirkulationssystems zu einem Spitzenbereich der Vorrichtung führt, um dessen Kühlung durch Verdampfen des kryogenen Stoffes zu bewirken, und kryogenen Stoff durch den zweiten Weg des inneren Zirkulationssystems entfernt, die Zufuhr von kryogener Flüssigkeit unterbricht, wenn der chirurgische Eingriff abgeschlossen ist, und ein auf eine vorbestimmte Temperatur erhitztes Inertgas durch erste und zweite Wege des inneren Zirkulationssystems führt, um ein Auftauen der Vorrichtung zu bewirken, **dadurch gekennzeichnet**, daß das durch die ersten und zweiten Wege zirkulierte Inertgas dem inneren Zirkulationssystem durch den ersten Weg zugeführt und aus dem inneren Zirkulationssystem durch den zweiten Weg entfernt wird.

2. Verfahren nach Anspruch 1, bei dem das Inertgas Stickstoff ist.

3. Verfahren nach Anspruch 1 oder Anspruch 2, bei dem das Inertgas der Vorrichtung über eine Wärmetauschanordnung (44) zugeführt wird.

4. Verfahren nach einem der vorausgehenden Ansprüche, bei dem die Vorrichtung eine kryochirurgische Sonde umfaßt.

5. Verfahren nach Anspruch 4, bei dem das Inertgas der Sondenspitze über ein Zuführungsrohr zugeführt wird, das entlang der Sondenachse liegt, und über einen Ringschlitz in dem das Zuführungsrohr umgebenden Sondengehäuse zurückgeführt wird.

6. Verfahren nach Ansprpuch 1, 2, 3, 4 oder 5, bei dem der kryogene Stoff flüssiger Stickstoff ist.

7. Vorrichtung nach einem der vorausgehenden Ansprüche, bei der die kryochirurgische Vorrichtung mehrere kryochirurgische Sonden (10, 12, 14, 16) umfaßt, von denen jede ein jeweiliges inneres Zirkulationssystem mit einem ersten Weg und einem zweiten Weg umfaßt, indem man für jede kryochirurgische Sonde die Zuführung von kryogener Flüssigkeit zu dem inneren Zirkulationssystem jener Sonde unabhängig steuert und für jede kryochirurgische Sonde, nachdem die Zuführung von kryogenem Stoff zu der Sonde unterbrochen wurde, die Zuführung von erhitztem Inertgas durch das innere Zirkulationssystem der Sonde unabhängig steuert.

8. Kryochirurgische Vorrichtung mit
einer kryochirurgischen Einrichtung (10, 12, 14, 16) mit einem inneren Zirkulationssystem zur Aufnahme von kryogenem Stoff, wobei das innere Zirulationssystem einen ersten Weg und einen zweiten Weg einschließt,
einer Einrichtung für die Zuführung von kryogener Flüssigkeit (28, 20, 22, 24, 26), um kryogene Flüssigkeit durch den ersten Weg des inneren Zirkulationssystems zu einem Spitzenbereich der Einrichtung zu führen und so den Spitzenbereich durch Verdampfung der kryogenen Flüssigkeit zu kühlen,
einer Abzugsleitung zur Aufnahme des abgeführten kryogenen Stoffes durch den zweiten Weg des inneren Zirkulationssystems und
einer Inertgaszuführungseinrichtung (40, 42, 44, 20, 22, 24, 26) zum Zuführen eines erhitzten Inertgases mit einer vorbestimmten Temperatur, nachdem die Zuführung von kryogenem Stoff am Ende des chirurgischen Eingriffs unterbrochen wurde, zu dem inneren Zirkulationssystem, um die kryochirurgische Einrichtung aufzutauen, **dadurch gekennzeichnet**, daß die Inertgaszuführungseinrichtung so gekoppelt ist, daß das Inertgas durch den ersten Weg des inneren Zirkulationssystems zugeführt wird.

9. Vorrichtung nach Anspruch 8, bei der die Inertgaszuführungseinrichtung einen Wärmetauscher (44) zum Erwärmen des Inertgases umfaßt, wenn es von einer Quelle (40) des Inertgases zu der kryochirurgischen Einrichtung führt.

10. Vorrichtung nach Anspruch 8 oder 9, bei der die kryochirurgische Einrichtung eine kryochirurgische Sonde (10, 12, 14, 16) umfaßt.

11. Vorrichtung nach Anspruch 8, 9 oder 10 mit mehreren kryochirurgischen Sonden (10, 12, 14, 16), von denen jede ein inneres Zirkulationssystem mit einem ersten Weg, mit welchem die Zuführungseinrichtung für kryogene Flüssigkeit und die Inertgaszuführungseinrichtung gekoppelt sind, und einem zweiten Weg, der mit der Abzugsleitung gekoppelt ist, hat.

## Revendications

1. Procédé de refroidissement et de dégel d'un appareil cryochirurgical (10, 12, 14, 16) comportant un système de circulation interne pour recevoir un cryogène, le système de circulation interne comprenant une première voie et une seconde voie, le procédé comprenant les opérations consistant à :
distribuer un cryogène liquide via la première voie du système de circulation interne jusqu'à une région d'extrémité de l'appareil pour la refroidir par vaporisation du cryogène, et extraire le cryogène via la seconde voie du système de circulation interne ;
interrompre la distribution de cryogène liquide lorsque la chirurgie est terminée ; et
faire circuler un gaz inerte chauffé à une température prédéterminée via la première voie et la seconde voie du système de circulation interne pour dégeler l'appareil ; caractérisé en ce que :
le gaz inerte amené à circuler via la première voie et la seconde voie est distribué jusqu'au système de circulation interne via la première voie, et est extrait du système de circulation interne via la seconde voie.

2. Procédé selon la revendication 1, dans lequel le gaz inerte est de l'azote.

3. Procédé selon la revendication 1 ou la revendication 2, dans lequel le gaz inerte est distribué jusqu'à l'appareil via un dispositif échangeur de chaleur (44).

4. Procédé selon l'une quelconque des revendications précédentes, dans lequel l'appareil comprend une sonde cryochirurgicale.

5. Procédé selon la revendication 4, dans lequel le gaz inerte est distribué jusqu'à l'extrémité de la sonde via un tube de distribution situé le long de l'axe de la sonde et est ramené via un espace annulaire au sein du boîtier de la sonde entourant le tube de distribution.

6. Procédé selon la revendication 1, 2, 3, 4, ou 5, dans lequel le cryogène est de l'azote liquide.

7. Procédé selon l'une quelconque des revendications précédentes, dans lequel l'appareil cryochirurgical comprend une pluralité de sondes cryochirurgicales (10, 12, 14, 16) comportant chacune un système de circulation interne respectif avec une première voie et une seconde voie, dans lequel le procédé comprend la commande indépendante, pour chaque sonde cryochirurgicale, de ladite distribution de cryogène liquide jusqu'au système de circulation interne de ladite sonde, et la commande indépendante, pour chaque sonde cryochirurgicale une fois que la distribution de cryogène jusqu'à ladite sonde a été interrompue, de ladite distribution de gaz inerte chauffé via le système de circulation interne de la sonde.

8. Appareil cryochirurgical comprenant :
un appareil cryochirurgical (10, 12, 14, 16) comportant un système de circulation interne pour recevoir un cryogène, le système de circulation interne comprenant une première voie et une seconde voie ;
un moyen de distribution de cryogène liquide (28, 20, 22, 24, 26) pour distribuer un cryogène liquide via la première voie du système de circulation interne jusqu'à une région d'extrémité de l'appareil pour refroidir la région d'extrémité par vaporisation du cryogène liquide ;
une conduite d'échappement pour recevoir le cryogène déchargé via la seconde voie du système de circulation interne ; et :
un moyen de distribution de gaz inerte (40, 42, 44, 20, 22, 24, 26) pour distribuer jusqu'au système de circulation interne, une fois que la distribution de cryogène a été interrompue à la fin de la chirurgie, un gaz inerte chauffé à une température prédéterminée pour dégeler l'appareil cryochirurgical ; caractérisé en ce que :
le moyen de distribution de gaz inerte est couplé pour distribuer le gaz inerte via la première voie du système de circulation interne.

9. Appareil selon la revendication 8, dans lequel le moyen de distribution de gaz inerte comprend un échangeur de chaleur (44) pour chauffer le gaz inerte lorsqu'il est distribué depuis une source (40) du gaz inerte jusqu'à l'appareil cryochirurgical.

10. Appareil selon la revendication 8 ou 9, dans lequel l'appareil cryochirurgical comprend une sonde cryochirurgicale (10, 12, 14, 16).

11. Appareil selon la revendication 8, 9 ou 10, comprenant une pluralité de sondes cryochirurgicales (10, 12, 14, 16) comportant chacune un système de circulation interne avec une première voie à laquelle le moyen de distribution de cryogène liquide et le moyen de distribution de gaz inerte sont couplés, et une seconde voie à laquelle ladite conduite d'échappement est couplée.
